# EUROPÄISCHE PATENTSCHRIFT

(11) **EP 0 379 605 B1**
(45) Veröffentlichungstag und Bekanntmachung des Hinweises auf die Patenterteilung: **30.03.1994**
(21) Anmeldenummer: 89101141.3
(22) Anmeldetag: 23.01.1989
(51) Int. Cl.: A61B 17/22

(54) **Medizinisches Gerät mit einem motorisch verstellbaren Geräteteil**
Medical device with a motor adjustable part
Appareil médical dont une partie est ajustable par moteur

(43) Veröffentlichungstag der Anmeldung: 01.08.1990
(73) Patentinhaber: SIEMENS AKTIENGESELLSCHAFT, 80333 München (DE)
(72) Erfinder: Grübl, Max, D-8520 Erlangen (DE); Schweiger, Hans-Jürgen, D-8520 Erlangen (DE)

(56) Entgegenhaltungen:
- EP-A- 0 048 978
- DE-A- 3 343 924
- DE-A- 3 608 877
- US-A- 4 610 249

## Beschreibung

Die Erfindung betrifft ein medizinisches Gerät gemäß dem ersten Teil des Patentanspruches 1.

Ein medizinisches Gerät dieser Art kann z.B. ein Stoßwellenkopf zum Zertrümmern von Konkrementen im Körper eines Lebewesens sein, der auf seiner Applikationsseite von einer flexiblen Membran zum Ankoppeln am Patienten abgeschlossen ist und gegenüberliegend eine Stoßwellenquelle aufweist, wobei der Raum zwischen Stoßwellenquelle und Membran mit einer Koppelflüssigkeit gefüllt ist und wobei im Stoßwellenkopf ein in axialer Richtung verstellbares Ultraschallsystem für die Ultraschallortung der Konkremente vorgesehen ist.

Mit einem Stoßwellenkopf dieser Art wird in der Weise gearbeitet, daß das Ultraschallsystem zur Ortung der Konkremente bei am Patienten angelegter Membran zunächst soweit motorisch in Richtung auf den Patienten verstellt wird, bis es an der Innenseite der Membran anstößt, also ebenfalls in Kontakt mit dem Patienten steht. Ist das Konkrement geortet, so wird das Ultraschallsystem in axialer Richtung nach rückwärts verstellt, so daß es bei der anschließenden Erzeugung von Stoßwellen zur Zertrümmerung des georteten Konkrementes nicht stört.

Bei einem medizinischen Gerät der eingangs genannten Art, insbesondere bei dem beschriebenen Stoßwellenkopf, muß sichergestellt werden, daß der verstellbar gelagerte Geräteteil keine unzulässig hohen Kräfte auf den Patienten ausübt.

Durch die EP-A-0 048 978 ist eine Tragvorrichtung für einen Patiententisch, der mit Hilfe einer Arbeitsmutter durch Drehen einer Spindel höhenverstellbar ist, bekannt. Eine Hilfsmutter fängt das Stativ für den Patientenlagerungstisch auf, wenn die Arbeitsmutter versagt. Die Kraft, bei der dieser Vorgang eintritt, ist durch das Versagen der Arbeitsmutter festgelegt und damit nicht definiert.

Der Erfindung liegt demgemäß die Aufgabe zugrunde, ein medizinisches Gerät gemäß dem ersten Teil des Patentanspruches 1 so auszubilden, daß die vom verstellbar gelagerten Geräteteil auf den Patienten ausgeübte Kraft auf ein vorbestimmtes Maß, also definiert begrenzt ist.

Diese Aufgabe ist erfindungsgemäß gelöst durch die Merkmale des zweiten Teiles des Patentanspruches 1. Die das verstellbare Geräteteil antreibende Mutter ist demgemäß durch eine vorbestimmte Kraft auf diesen Geräteteil auf der sie antreibenden Spindel verstellbar, so daß die Kraft auf den Patienten, gegen den das Geräteteil bis zum Anlegen an seiner Oberfläche verstellt wird, begrenzt ist.

Die Erfindung eignet sich in besonders zweckmäßiger Weise zur Anwendung bei einem Stoßwellenkopf der geschilderten Art, wobei das verstellbare Geräteteil vom Ultraschallsystem gebildet ist.

Die Erfindung ist nachfolgend anhand eines in der Zeichnung dargestellten Ausführungsbeispiels näher erläutert. Es zeigen:
- Fig. 1: einen Stoßwellenkopf nach der Erfindung, und
- Fig. 2: die für die Erfindung wesentlichen Teile des Stoßwellenkopfes gemäß Fig. 1.

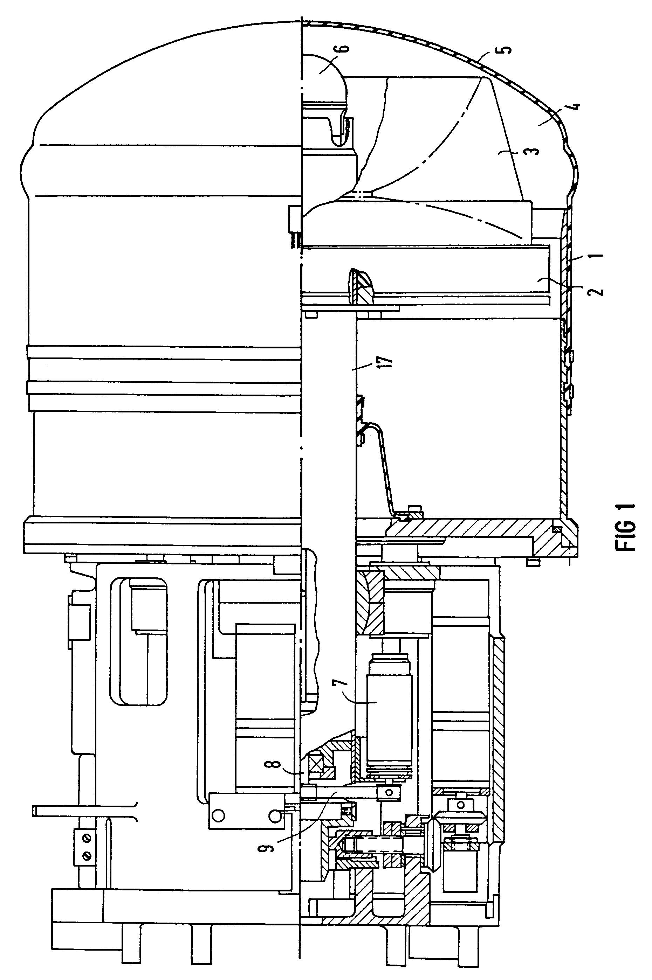

Der in der Fig. 1 dargestellte Stoßwellenkopf weist in einem Gehäuse 1 eine Stoßwellenquelle 2 auf, die Stoßwellen über eine akustische Linse 3 und einen mit einer Koppelflüssigkeit gefüllten Raum 4, welcher durch eine flexible Membran 5 abgeschlossen ist, auf einen Patienten richtet. Hierzu wird die Membran 5 an der Körperoberfläche des Patienten angelegt. Die Ortung der mit Hilfe der fokussierten Stoßwellen zu zertrümmernden Konkremente erfolgt durch ein axial im Stoßwellenkopf angeordnetes Ultraschallsystem 6, welches in axialer Richtung verstellbar ist. Hierzu ist ein Verstellmotor 7 vorgesehen, der eine in der Fig. 1 nur teilweise dargestellte Spindel 8 über einen Zahnriemen 9 dreht.

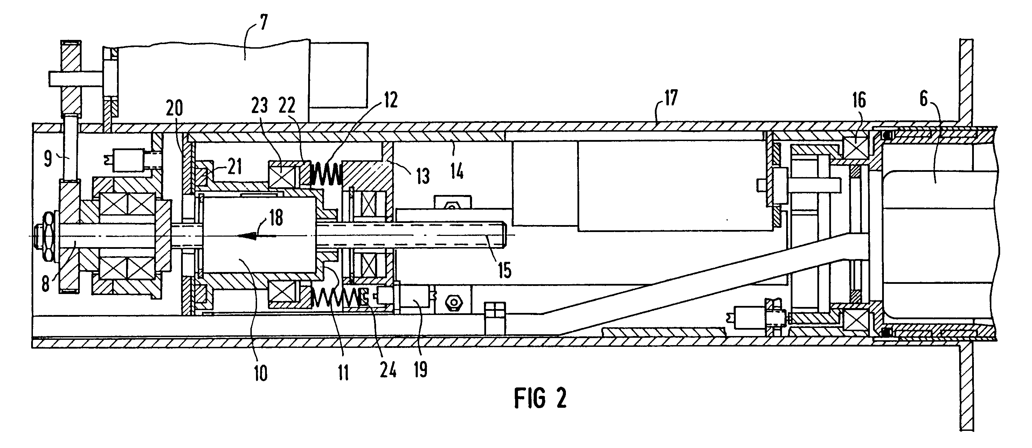

Die Fig. 2 zeigt, daß auf der Spindel 8 eine Mutter 10 gleitet, welche mit einem Gehäuse 11 verbunden ist, das sich über Federn 12 gegen ein Lager 13 abstützt. Das Lager 13 ist mit einem Rohr 14 fest verbunden, welches das Ultraschallsystem 6 in axialer Richtung unverschiebbar trägt. Das Ultraschallsystem 6 ist jedoch mit dem Rohr 14 um die gemeinsame Achse 15 drehbar verbunden (Kugellager 16). Das Rohr 14 ist in einem gerätfesten Rohr 17 in axialer Richtung verschiebbar. Diese Verschiebung erfolgt beim Drehen der Spindel 8, da die Spindel 8 in axialer Richtung fest ist, so daß dann die Mutter 10 auf der Spindel 8 verstellt wird. Sie nimmt über das Gehäuse 11 und das Lager 13 das Rohr 14 mit.

Wird das Ultraschallsystem 6 über das Rohr 14 soweit nach vorne verstellt, daß es an der Membran 5 anliegt und damit im Kontakt mit der Körperoberfläche des Patienten steht, so kann die Ultraschallortung von Konkrementen erfolgen. Hierzu kann das Ultraschallsystem 6 um die Achse 15 gedreht werden. Falls sich der Patient bewegt oder aus anderen Gründen die Kraft zwischen dem Ultraschallsystem 6 und dem Patienten zu groß wird, werden die Federn 12 über das Rohr 14 und das Lager 13 zusammengedrückt. Dadurch löst sich der Reibschluß zwischen der mit dem Rohr 14 verbundenen Scheibe 20 und dem mit dem Gehäuse 11 verbundenen Ring 21. Die Mutter 10 dreht sich mit ihrem Gehäuse 11 in dem Lager 23 um das Lager 22, so daß sie mit dem Rohr 14 und dem Ultraschallsystem 6 entgegen der durch die Drehrichtung der Spindel 8 vorgegebenen Verstellrichtung nach rückwärts verstellt wird. Die Kraft, die das Ultraschallsystem 6 auf den Patienten bzw. auf die Membran 5 ausübt, kann demgemäß einen vorbestimmten Wert nicht überschreiten. Die Steigung des Gewindes der Spindel 8 und der Mutter 10 ist so gewählt, daß die geschilderte Verstellung der Mutter 10 in Richtung des Pfeiles 18 durch eine entsprechende Kraft auf das Ultraschallsystem 6 bei sich drehender Spindel 8 möglich ist. Der Verstellweg der Mutter 10 mit den damit verstellten Teilen in Richtung des Pfeils 18 wird durch einen Endschalter 19 begrenzt, der betätigt wird, wenn ein mit einer der Federn 12 verbundener Betätiger 24 dort anstößt. Der Endschalter 19 setzt dann den Motor 7 still.

## Patentansprüche

1. Medizinisches Gerät, welches einen motorisch verstellbar gelagerten Geräteteil (6) aufweist, der mit einer Mutter (10) verbunden ist, welche auf einer motorisch gedrehten Spindel (8) läuft, wobei das Gewinde derart gewählt ist, daß die Mutter (10) beim Überschreiten einer bestimmten axialen Kraft auf der Spindel (8) entgegen der durch die Spindel (8) vorgegebenen Verstellrichtung verstellt wird, **dadurch gekennzeichnet,**
daß die Mutter (10) bei einer vorbestimmten axialen Druckkraft auf der Spindel (8) verstellt wird, daß hierzu die Gewindesteigung entsprechend gewählt ist und daß zwischen dem Geräteteil (6) und der Mutter (10) Federn (12) zur Festlegung der vorbestimmten axialen Druckkraft vorhanden sind.

2. Medizinisches Gerät nach Anspruch 1, **dadurch gekennzeichnet,** daß der verstellbare Geräteteil (6) ein Ultraschallsystem ist, das in einem Stoßwellenkopf zum Zertrümmern von Konkrementen im Körper eines Patienten in axialer Richtung verstellbar ist, wobei der Stoßwellenkopf auf seiner Applikationsseite eine flexible Membran (5) zum Ankoppeln am Patienten und auf seiner anderen Seite eine Stoßwellenquelle (2) aufweist und der Raum (4) zwischen Stoßwellenquelle (2) und Membran (5) mit einer Koppelflüssigkeit gefüllt ist.

## Claims

1. Medical apparatus which has a motor-driven adjustably mounted apparatus part (6) which is connected to a nut (10) which travels on a motor-rotated spindle (8), wherein the thread is selected so that when a specific axial force on the spindle (8) is exceeded, the nut (10) is adjusted counter to the adjustment direction predetermined by the spindle (8), characterised in that the nut (10) is adjusted when there is a predetermined axial pressure force on the spindle (8), in that in addition the thread pitch is appropriately selected, and in that there are springs (12) between the apparatus part (6) and the nut (10) for fixing the predetermined axial pressure force.

2. Medical apparatus according to claim 1, characterised in that the adjustable apparatus part (6) is an ultrasonic system which can be adjusted in the axial direction for disintegrating concrements in the body of a patient in a shock wave head, wherein the shock wave head has a flexible diaphragm (5) on its application side for coupling to the patient, and on its other side has a shock wave source (2), and the space (4) between the shock wave source (2) and the diaphragm (5) is filled with a coupling fluid.

## Revendications

1. Appareil médical qui comporte une partie (6) montée de manière à être réglable à l'aide d'un moteur et qui est raccordée à un écrou (10), qui se déplace sur une broche (8) entraînée en rotation par un moteur, le filetage étant choisi de telle sorte que, lors du dépassement d'une force axiale déterminée, l'écrou (10) est déplacé sur la broche (8) en sens opposé du sens de déplacement prédéterminé par la broche (8), caractérisé par le fait que l'écrou (10) est déplacé sur la broche (8), dans le cas d'une force de pression axiale prédéterminée, qu'à cet effet le pas du filetage est choisi de façon correspondante et que des ressorts (12) servant à déterminer la force de pression axiale prédéterminée sont présents entre la partie (6) de l'appareil et l'écrou (10).

2. Appareil médical suivant la revendication 1, caractérisé par le fait que la partie réglable (6) de l'appareil est un système à ultrasons qui est déplaçable dans la direction axiale à l'intérieur d'une tête de production d'ondes de choc servant à fragmenter des concrétions à l'intérieur du corps d'un patient, la tête de production d'ondes de choc possédant, sur son côté d'application, une membrane flexible (5) permettant un couplage avec le patient, et sur son autre côté, une source d'ondes de choc (2), et que l'espace (4) présent entre la source d'ondes de choc (2) et la membrane (5) est rempli par un liquide de couplage.
